# EUROPEAN PATENT SPECIFICATION

(11) **EP 1 259 157 B1**
(45) Date of publication and mention of the grant of the patent: **11.06.2008**
(21) Application number: 01910113.8
(22) Date of filing: 01.03.2001
(51) Int. Cl.: A61B 5/02, A61B 5/103, A61B 5/022

(54) **METHOD AND APPARATUS FOR THE NON-INVASIVE DETECTION OF PARTICULAR SLEEP-STATE CONDITIONS BY MONITORING THE PERIPHERAL VASCULAR SYSTEM**
VERFAHREN UND VORRICHTUNG ZUR NICHT-INVASIVEN BESTIMMUNG VON SCHLAFZUSTÄNDEN MITTELS ÜBERWACHUNG DES PERIPHEREN VASKULARSYSTEMS
TECHNIQUE ET APPAREIL CORRESPONDANT DE DETECTION NON EFFRACTIVE DE CONDITIONS PARTICULIERES DE L'ETAT DE SOMMEIL PAR SURVEILLANCE DU SYSTEME VASCULAIRE PERIPHERIQUE

(30) Priority: 02.03.2000 US 186358 P; 19.06.2000 US 212648 P; 16.10.2000 US 240079 P
(43) Date of publication of application: 27.11.2002
(73) Proprietor: Itamar Medical Ltd, 38900 Caesarea (IL)
(72) Inventor: LAVIE, Peretz, 34981 Haifa (IL); SCHNALL, Robert, P., 27201 Kiryat Bialik (IL); SHEFFY, Jacob, 34401 Haifa (IL)
(74) Representative: Schmitz, Jean-Marie
(86) International application number: PCT/IL2001/000199
(87) International publication number: WO 2001/064101

(56) References cited:
- WO-A-98/04182
- US-A- 5 101 831
- US-A- 5 398 682
- US-A- 5 917 415

## Description

### FIELD AND BACKGROUND OF THE INVENTION

The present invention relates to a method and apparatus for the non-invasive detection of certain medical conditions, particularly certain sleep-state conditions, in an individual by monitoring the peripheral vascular system of the individual.

PCT Applications No. PCT/IL97/00249published February 5, 1998 (International Publication No. WO 98/04182); No. PCT/IL99/00292 published December 16, 1999 (WO 99/63884), No. PCT/IL00/00307 published December 14, 2000 (International Publication No. WO 00/74551), No. PCT/IL00/00403 published January 18, 2001 (WO 01/03569), describe non-invasive devices for measuring blood pressure and for detecting various medical conditions, including: myocardial ischemia, certain sleep state conditions, endothelial dysfunction (ED), and stress-induced myocardial ischemia. In the preferred embodiments described in those applications, the described non-invasive devices used volume-measuring sensors and optical sensors for measuring changes in the peripheral arterial bed volume of the individual, which changes were translated to changes in the peripheral arterial tone.

The present application is directed to detecting certain sleep-state conditions, particularly sleep disordered breathing in an individual.

The broad area of sleep disordered breathing encompasses a number of recognized abnormal conditions. including: the obstructive and central sleep apnea syndrome, which results in complete cessations of breathing that occur repeatedly during sleep; obstructive hypopneas, which results in partial upper airway obstruction and reduced ventilation; and the upper airway resistance syndrome (UARS), which results in subtle respiratory changes even though the airflow may appear to be normal. All the foregoing conditions produce frequent awakenings and sleep fragmentation which result in impaired sleep quality and daytime functioning.

Even with the comprehensive battery of measurements used in laboratory based polysomnographic evaluations, the diagnosis of UARS is extremely problematical due to the difficulty in visibly scoring the subtle respiratory changes. [Guilleminault C, Stoohs R, Clark A, Cetel M and Maistros P, "A Cause of Excessive Daytime Sleepiness. The Upper Airway Resistance Syndrome", Chest 104:781-787 (1993)]. A proper diagnosis of this syndrome necessitates the insertion of an esophageal balloon to measure the patient's intra-thoracic pressure. This technique causes great inconvenience and is not well tolerated.

A possible measurable parameter for aiding in the diagnosis of UARS may be the occurrence of frequent cortical or autonomic arousals during sleep. This may be marked by bursts of alpha electro-encephalographic (EEG) activity which signify awakening, or by increased sympathetic activation. In many cases, however, there is only increased sympathetic activity with no evidence of cortical arousal in the EEG.

A simple and robust method and apparatus, capable of being used outside the confines of the sleep laboratory for monitoring the sleep state condition, and particularly for marking arousals, would be a very important diagnostic tool in the identification of what might otherwise be an unrecognizable disease state.

### BRIEF SUMMARY OF THE PRESENT INVENTION

According to one aspect of the present invention, there is provided a method of monitoring an individual for acquiring data useful with other data for determining the occurrence of a particular condition during the sleep state of the individual, comprising: applying an external probe to an external surface at a peripheral body location on the individual's body for monitoring the peripheral vascular bed volume of the individual at the peripheral body location by applying a predetermined pressure field to the distal end of the peripheral body location including Its distal-most extremity so as to prevent the occurrence of venous pooling within and distal to the peripheral body location, and thereby to produce an output signal from the probe corresponding to changes in the peripheral arterial bed volume at the peripheral body location; while the individual is in a sleep state, utilizing the probe to monitor changes in the peripheral body location and to produce an output corresponding thereto; and determining the sleep state condition of the individual according to the output of thermoelectric cooling device external probe; wherein the sleep state condition of the individual is determined when a predetermined attenuation, and a predetermined time interval between attenuations, are detected in the output of said external probe.

Preferably, the external probe used is one of the several non-invasive finger-probes described in the above-cited PCT applications for monitoring the peripheral vascular bed volume of the individual, and for translating the measurements to changes in peripheral arterial tone. Particularly described were volume-measuring probes and light-measuring probes applied to a finger (or toe) of the individual. Such probes were found to confer numerous advantages in monitoring the individual's vascular system for changes in the peripheral arterial bed volume, particularly in the following respects:
1) By applying near diastolic pressure over the surface of the finger, which is transmitted to the arteries within the finger, they reduce the transmural pressure within those arteries, thereby freeing the arterial walls of tension and increasing their compliance allowing them to move more freely;
2) By providing such pressure, they are able to prevent the pooling of venous blood in the measured part of the finger, thereby avoiding the occurrence of venous distention and possible reflex arterial constriction as a result of the venous distention;
3) By providing a contiguous buffer region proximal to the measurement site, they are able to reduce the effects of retrograde venous pressure perturbations and to extend the effective boundary of the pressure field in the measurement portion of the probe.

The foregoing features of the finger probes described in the above-cited patent applications, which are also present in the finger probes described below, have been found to enhance their performance for the many described uses of the probe. While such probes remain the preferred ones due to these advantageous characteristics, it will be appreciated that the invention described below can also be implemented by using other peripheral vascular bed volume monitoring devices for detecting the specific markers to be described below. Examples of other types of devices for monitoring the changes in the peripheral vascular bed volume include; segmental plethysmographs, circumferential strain gage devices, optical plethysmographs, Doppler or laser Doppler sensors,; isotope washout devices, thermal washout devices, electromagnetic devices, and any other devices which are affected by a change in the geometry of the finger (or other peripheral body part, e.g. toe, ear-lobe) in response to blood volume changes.

Examples of different sleep state conditions detected in accordance with the invention described below include: arousals during the sleep state, apneas, hypopneas, UARS events, a Cheynes-Stokes breathing pattern, periodic leg movements syndrome (PLMS) and rapid eye movement sleep state (REM).

According to another aspect of the present invention, there is provided apparatus for monitoring an individual to detect the occurrence of a particular condition during the sleep state of the individual, comprising: an external probe to be applied to an external surface at a peripheral location on the individual's body for monitoring the peripheral vascular bed volume of the individual at the peripheral body location; the external probe including a pressure applicator for applying a predetermined pressure field to the peripheral body location including its distal-most extremity to reduce venous pooling and thereby to produce an output signal from the probe corresponding to changes in the peripheral arterial bed volume at the peripheral body location; and a signaling device for producing a signal indicating the predetermined sleep state condition when a predetermined change in the output of the probe is detected; wherein said signaling device produces a signal indicating said sleep state condition when the output of said probe detects a predetermined attenuation, and a predetermined time interval between attenuations.

According to further features in the described preferred embodiments, the apparatus may further include a sleep-wake detector for detecting the sleep/wake state of the individual; a pulse oximeter for monitoring the blood oxygen saturation level of the individual and which can be either added as a separate detector, or can be incorporated within the probe which detects the peripheral volume changes; an oral-nasal airflow sensor for monitoring the oral/nasal airflow of the individual; and/or an electrocardiographic (ECG) electrode depole for permitting aspects of the patient's ECG to be determined. Such detectors may produce outputs which may be used by the signaling device with the external probe output and/or with other sensors commonly included in polysomnographs, to determine the sleep state condition of the individual.

Further features and advantages of the invention will be apparent from the description below.

### BRIEF DESCRIPTION OF THE DRAWINGS

The invention is herein described, by way of example only, with reference to the accompanying drawings, wherein:
Fig. 1 illustrates one form of apparatus, as described in the above-cited PCT applications, that may be used to implement the present invention as described below;
Fig. 2 illustrates another finger-probe, including an optical sensor, which may be used in the apparatus of Fig. 1;
Fig. 3 is a block diagram illustrating one form of overall system constructed in accordance with the present invention particularly useful for monitoring various conditions during the sleep state of the individual;
Fig. 4 is a block diagram illustrating the incorporation of a finger-probe in accordance with the present invention as one input in a known polysomnograph system;
Fig. 5 is illustrates the finger-probe output waveform of a sleeping individual compared to the individual's EEG waveform showing sleep arousals;
Fig. 6 illustrates the finger-probe output waveform of a sleeping individual compared to the individual's EEG spectrum, also showing sleep arousals;
Fig. 7 illustrates the finger-probe output waveform of a sleeping individual compared to the individual's breathing pattern showing Cheyne-Stokes breathing;
Figs. 8a and 8b illustrate the finger-probe output waveforms of a sleeping individual compared to other sensor waveforms of the individual showing both apnea events and sleep arousals;
Fig. 9 is a scattergraph showing how the finger-probe output can be used in the known Detrended Fluctutation Analysis (DFA) method for the detection of congestive heart failure (CHF); and
Fig. 10 is a scattergraph showing how the finger-probe output can be used in the known DFA method for the detection of rapid eye movement (REM) stage sleeping.

### DESCRIPTION OF PREFERRED EMBODIMENTS

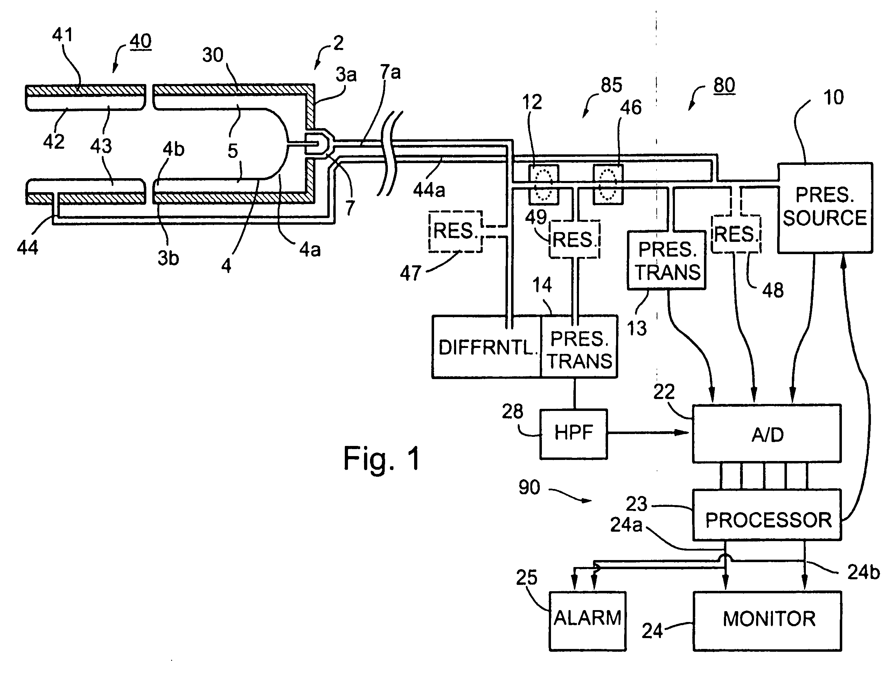

Fig. 1 illustrates apparatus corresponding to that described in the above-cited PCT applications including a finger probe, generally designated 2. Finger-probe 2 comprises a thimble-shaped end cap 30 and a pressure cuff 40 connected to a pneumatic system, generally designated 80, which is in turn connected to a processing system, generally designated 90. The pneumatic system 80 includes a pressure source 10 connected to a pneumatic tubing system, generally designated 85. The tubing system includes tubes 7a and 44a, which deliver the pressure from the pressure source to the finger-probe 2, and electronic solenoid valves 12 and 46, which can be controlled by the processor 23 to be described later.

The pneumatic system 80 further includes a pressure transducer 13 for monitoring the pressure applied by source 10, and a differential pressure transducer 14 measuring the difference between the variable pressure in the finger-probe chambers and the constant pressure existing between valves 12 and 46. Optionally, the pneumatic tube 85 may be further provided with reservoirs 47, 48 and 49.

The processing system 90 includes an A/D converter 22, a processor 23, and a monitoring device, generally indicated as monitor 24 and alarm 25. The processing system is responsible for controlling the operation source 10 and solenoids of valves 12 and 46, and also processes the detected signals to provide a decipherable output

In order to perform a diagnostic procedure, the valves 12 and 46 are first open and the chambers 5 and 43 of the finger-probe are evacuated to allow the patient to insert a finger into the probe. Then, the pressure is raised to a pressure which is sufficient to unload the arterial walls and to prevent venous pooling. The pressure applied by source 10 is measured by a pressure transducer 13 upstream of valves 12 and 46. In the preferred embodiment, the pressure in the pneumatic compartments is automatically raised to 70 mm Hg.

At this point, valves 12 and 46 are closed, so that the pressure in the right chamber of pressure differential transducer 14 is kept constant. On the other hand, the pressure on the left chamber of transducer 14 varies depending on the pressure inside chamber 5 of the finger-probe 2. Notably, for detection of peripheral vasoconstriction, no calibration of the inventive device is necessary, since the measurement is comparative with the patient's own baseline results observed during the test.

Changes in the volume of the subject's finger which are due to arterial blood pressure pulse waves produce an expansion or contraction of chamber 5, and a corresponding decrease or increase in the gas pressure within chamber 5. Chamber 5 is connected via its port 7 and tube 7a to the pneumatic tubing 85. However, since valve 12 is closed, the pressure changes affect only the left chamber of differential-pressure sensor 14. The differential pressure sensor 14 detects these pressure changes and provides an output corresponding to the pressure changes.

The A/D converter 22 shown in Fig. 1 receives the analog outputs of pressure transducers 13 and 14, and converts them into digital form before introducing them into a CPU processor 23. The processor 23 processes the measured finger volume (or optical density) changes to produce output 24a of the volume measurements, and/or an output 24b of the changes in the volume measurements with respect to time. Either one or both measurements can be displayed on the monitor 24 as well as recorded in the memory.

If the displayed output 24 shows a change in the measured volume, indicating peripheral vasoconstriction, this will be immediately seen by the observer viewing monitor 24.

The peak to trough amplitude of the signal is generally proportional to the arterial pulsatile volume changes, and will decrease or attenuate upon peripheral vasoconstriction. Therefore, when the system of Fig. 1 is used for detecting peripheral vasoconstriction, the observer would be interested in relative changes of the amplitude of the trough to peak values, as opposed to the absolute values of the pressure.

As previously described, it is preferable that the finger-probe include an annular pressure cuff 40 coaxial with and contiguous to the end cap 30, on the proximal (heart) side of the device. The main purpose of the pressure cuff is to extend the boundary of the constant pressure field beyond the borders of the sensing probe, so as to avoid edge effects. Chamber 43 of the pressure cuff is also filled with a pressurized gas via a port 44; however, solenoid valve 46 isolates conduit 44 from transducer 14. Cuff 40 thus extends the static pressure field for a distance in the proximal (heart) direction from the site of measurement of the finger volume changes accompanying blood pressure waves. The annular pressure cuff 40 acts as a venous tourniquet which, together with the pressure field produced in the thimble-shaped end cap 30, prevents venous pooling in the distal end (particularly the most distal phalange) of the finger. It also substantially prevents uncontrolled venous back flow; and further, it partially unloads the wall tension of, but does not occlude, the arteries in the distal end of the finger when the finger is at heart level. While the pressure in the pressure cuff may differ from that in the sensing chambers 35, 36, it should not exceed it.

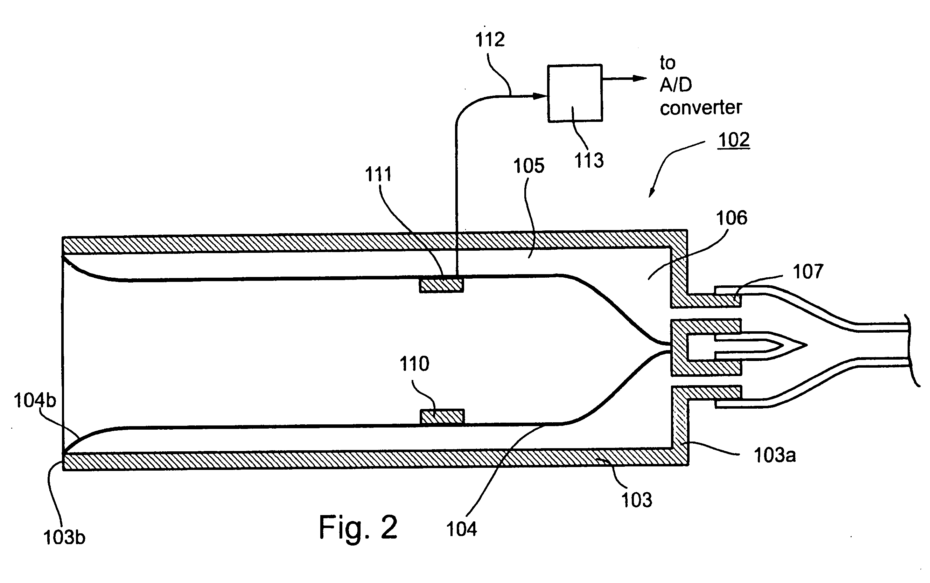

Fig. 2 illustrates an apparatus also described in the above-cited PCT applications. It is similar to that of Fig. 1 except that changes in the optical density are directly sensed and measured to provide a measurement of the changes in the finger accompanying the blood pressure waves. Except for the optical sensing elements the finger-probe illustrated in Fig. 2 is similarly constructed as described above with respect to Fig. 1. To facilitate understanding, the finger-probe in Fig. 2 is generally designated 102; and its elements generally corresponding to those described above in Fig. 1, particularly elements 3, 3a, 3b, 4, 4b, 5, 6 and 7, are identified by the same reference numerals except increased by "100", i.e., as elements 103, 103a, 103b, 104, 104b, 105, 106 and 107, respectively. The optical elements in Fig. 2 include a light source 110, and a light receiver 111.

Thus, in the apparatus illustrated in Fig. 2, chamber 105 is pressurized to a fixed predetermined value, as described above with respect to Fig. 1. In this case, however, the tubular diaphragm 104 defining chamber 105 is provided on one side with the light source 110, and on the opposite side with the light receiver 111, such that pulsatile blood volume changes in the finger received within the tubular diaphragm 104 will be detected as changes in optical density by the light receiver 111. This information is fed via conductors 112 to an amplifier circuit 113 where it is amplified and filtered, and then fed to the A/D converter (22, Fig. 1) for processing by the processor (23, Fig. 1) as described above.

In the arrangement illustrated in Fig. 2, the measurement site, namely the location of the light source 110 and light receiver 111, is considerably inward of the open end of the rigid casing 103 of the probe 102 which applies the static pressure field uniformly around the outer end of the finger, and therefore the annular pressure cuff (40, Fig. 1) need not be included. However, if it is desired to locate the light source and light collector doser to the open end of the rigid casing of the probe 102, the annular pressure cuff (corresponding to pressure cuff 40 in Fig. 1), may also be used in the system illustrated in Fig. 2.

Further details of such apparatus and various modifications thereof, as well as methods of using the apparatus for diagnosing various medical conditions, are described in the above-cited PCT Applications.

While the descriptions in the above-cited PCT applications focus mainly on measuring blood pressure and detecting myocardial ischemia, the method and apparatus of the present invention are directed primarily to monitoring various sleeping conditions of a subject, particularly the rapid eye movement (REM) sleep stage, sleep arousals, sleep apnea, sleep hypopnea, upper airways resistance, periodic leg movement syndrome, Cheyne-Stokes breathing, and congestive heart failure.

Sleep staging, in particular the determination of REM (Rapid Eye Movement) stage sleep, is a vital tool for diagnosing sleep disorders and numerous other conditions. During REM sleep, altered control of breathing occurs with greatly reduced chemosensitivity resulting in highly irregular breathing patterns and the greatest declines in blood oxygen saturation.

Changes in REM latency have been reported in a plethora of affective illnesses including endogenous depression, schizophrenia, anxiety disorders, obsessive-compulsive disorders, eating disorders as well as in narcolepsy, alcoholism, Alzheimer's disease and impotence. REM latency is important not only in the diagnosis of these conditions but also in therapy and follow up since it is a sensitive indicator of the patient's condition.

A robust association was found between REM stage sleep and the attenuation of the finger-probe output signal. This attenuation was of a substantial magnitude compared to the prior non REM period. Three representative examples showing the time-course of the probe-output signal and sleep hypnograms were shown in Fig. 21 of PCT/IL97/00249. It is important to note that the attenuation of the finger-probe output signal amplitude was not triggered by REM sleep, but appeared to be related to an ongoing cycle that was synchronized with the sleep stages cycle in such a way that the nadirs of this cycle coincided with REM sleep.

Currently, REM stage sleep is identified by polysomnography, which requires costly apparatus, considerable patient instrumentation and specialized staff. One simplified REM detector is the "night cap" disclosed in USA Patent No. 4,836,219 to Hobson et al. which relies on two channels of information to detect REM sleep; body movements and eye movements. However, this method requires substantial instrumentation which may be uncomfortable for the patient and detrimental to sleep. Another patented device (USA Patent No. 5,280,791 to Lavie) employs a heart rate variability method. However, this method requires demanding signal analysis, and may not be as reliable as the method utilizing the output signal of the finger-probe.

REM detection utilizing the above-described probe could be an extremely useful adjunct to existing ambulatory monitoring systems, since it yields important information with a minimum of patient instrumentation in a highly cost effective manner. It could be used to provide intensive, long term, follow up in the patient's own home, which would be a logistic impossibility in the sleep lab setting. The probe could be readily used in combination with oxygen saturation monitoring and ambulatory apnea screening. It could eliminate the need for subjective operator evaluation of sleep studies and the dependency on the specialized and expensive instruments needed for laboratory based sleep staging, such as EEG, EOG and EMG measurements.

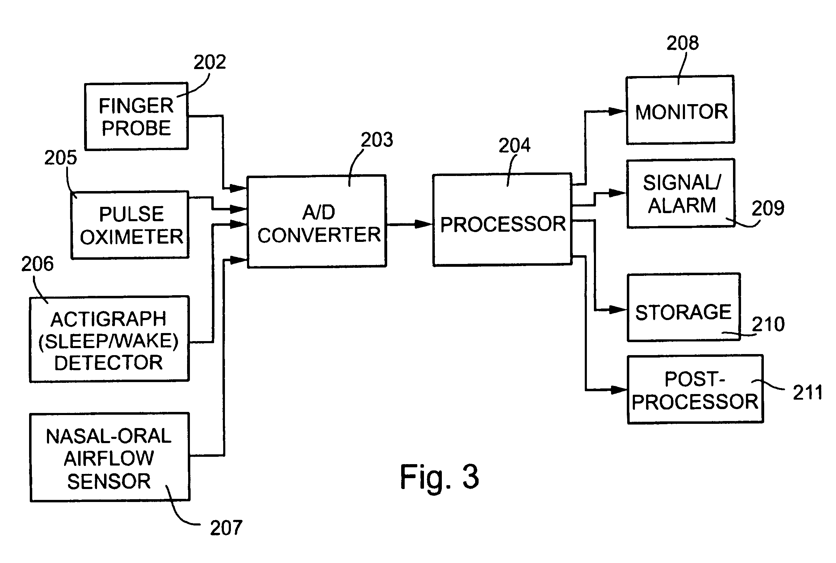

Fig. 3 illustrates an example of a set-up which may be used for performing these tests. In this illustrated set-up, the finger-probe 202 (e.g., of the construction described above in Fig. 1 or Fig. 2) provides one input, via an analog-digital converter 203, to a processor 204. The processor 204 includes additional inputs from a pulse oximeter 205, which measures the oxygen saturation level of the blood; from an actigraph 206, which serves as a sleep-wake detector; and from a nasal-oral airflow sensor 207, such as a thermistor, which measures the nasal-oral air flow of the sleeping patient; and from at least a single electrocardiograph electrode dipole for registering the patient's ECG.

The foregoing inputs are processed by processor 204, which produces outputs to a monitor 208, a signal and/or alarm unit 209, a storage device 210, a post-processor device 211.

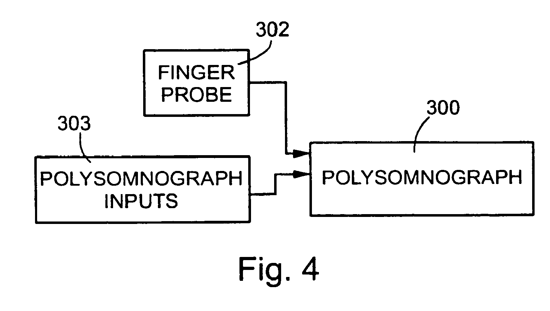

Fig. 4 illustrates another set-up including a conventional polysomnograph 300, which includes the output from the finger-probe 302 as one of the inputs, together with the other inputs 303 commonly provided in such apparatus.

During numerous highly controlled sleep studies in elite sleep laboratories, it was also found that there was a highly robust association between the appearance of alpha wave encephalographic activity signifying awakening during sleep, and attenuation of the finger-probe signal. This attenuation was of a substantial magnitude and was tightly linked to the appearance of the alpha wave activity, with a consistent time delay between the two phenomena.

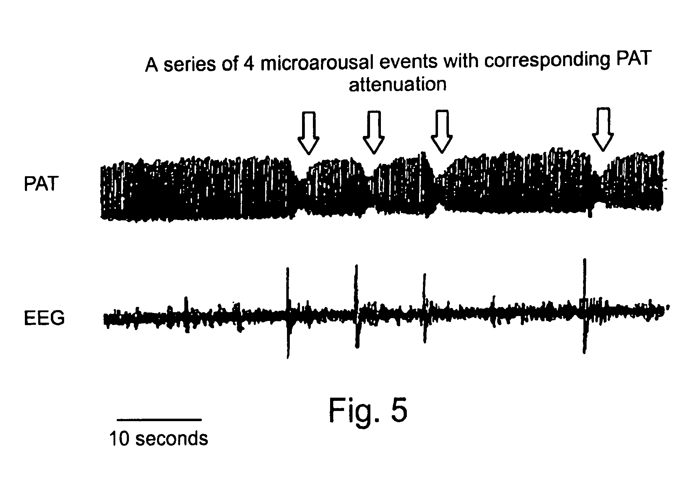

A representative example showing the time-course of the finger-probe signal and the patient's EEG signal associated with such alpha wave activity is shown in Fig. 5 illustrating a compressed record of a series of K-alpha events. These are typical microarousals, each comprised of a K complex with a tail of alpha EEG activity. These events are the hallmark of the upper airway resistance syndrome (UARS) and may therefore be used for detecting UARS.

It is important to note that the maximum attenuation of the finger-probe signal amplitude always occurred after the appearance of the alpha wave. The finger-probe signal may therefore also be used for detecting UARS.

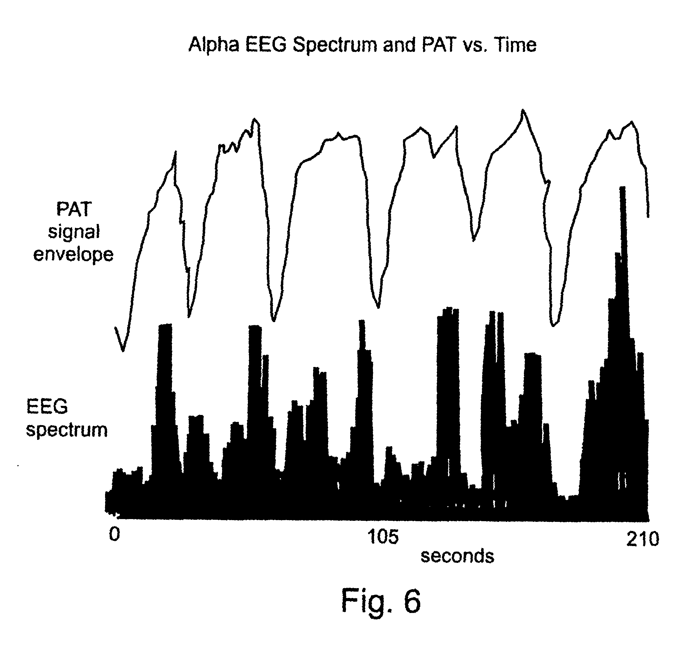

The same strong temporal linkage is clearly seen in the spectrum of the EEG signal which can also be considered as a marker of the alpha activity. This is shown in Fig. 6 wherein the upper waveform is the finger-probe output signal envelope, and the lower waveform is the EEG spectrum of the sleeping individual.

In addition to the highly consistent linkage between alpha activity and finger-probe output over time, it was found the attenuation of the finger-probe output reached its nadir (trough) several milliseconds after the peak alpha activity Thus, alpha activity leads the nadir attenuation. This delay has been consistently found in a large number of patients and normals and therefore can also be used as an indication of UARS.

An additional finding of diagnostic significance is that it is possible to distinguish between arousals related to the periodic leg movement syndrome (PLMS) and arousals related to disordered breathing events during sleep on the basis of the time interval between probe signal attenuation events. For example, the average time interval between probe signal attenuation events in PLMS is 24.8 seconds while respiratory related attenuation events related to hypopneas had a mean interval of 34.1 seconds. Based on the interval between attenuations it is possible to distinguish between the non-respiratory and respiratory related events with a very high degree of sensitivity of about 85%.

Brief arousal detection by the finger probe could be an extremely useful adjunct to existing ambulatory monitoring systems, since it yields important information with a minimum of patient instrumentation in a highly cost effective manner. Furthermore, adding the finger-probe signal to existing sleep laboratory recordings can aid in the rapid identification of brief arousals. Such a probe could also be used to provide intensive, long term, follow up in the patient's own home, as well as a means for assessing treatment effectiveness, which would be a logistic impossibility in the sleep lab setting.

Adding the probe to provide an input to the polysomnograph apparatus, as illustrated in Fig. 3 or 4, enables the probe to be readily used in combination with respiratory indices currently used for apnea screening, such as oro-nasal airflow and pulse oximetry. Such apparatus would better enable a differential diagnosis to be made between arousals related to obstructive sleep disordered breathing, non-obstructive sleep disordered breathing disorders, or arousal from sleep such as those occurring in the periodic leg movement syndrome (PLMS). Generally speaking, such a probe would enable UARS, and the other respiratory and non-respiratory conditions which are associated with arousal, to be identifiable both under the sleep laboratory environment, as well as under circumstances in which contemporary sleep apnea ambulatory screening methods would be unable to detect the condition. Also, the conventional arousals scoring which is EEG based, is insensitive to non-cortical arousals and suffers from large inter and intra scorers variability. The analysis of the suggested add on signal to the polysomnography, can be readily done automatically with no such variabilities and, as mentioned before, can add the detection of arousals having non-cortical indices.

In addition to the above described sleep related breathing disorders, there exists a different class of breathing disorders which are not obstructive in nature but which are in fact related to aberrant central nervous system control of breathing during sleep. The phase relationship between the probe output and the alpha waves, as well as the phase relationship between the probe output and the variations in oxygen saturation, can provide important information about the type of the apnea as well as the associated hemodynamic changes.

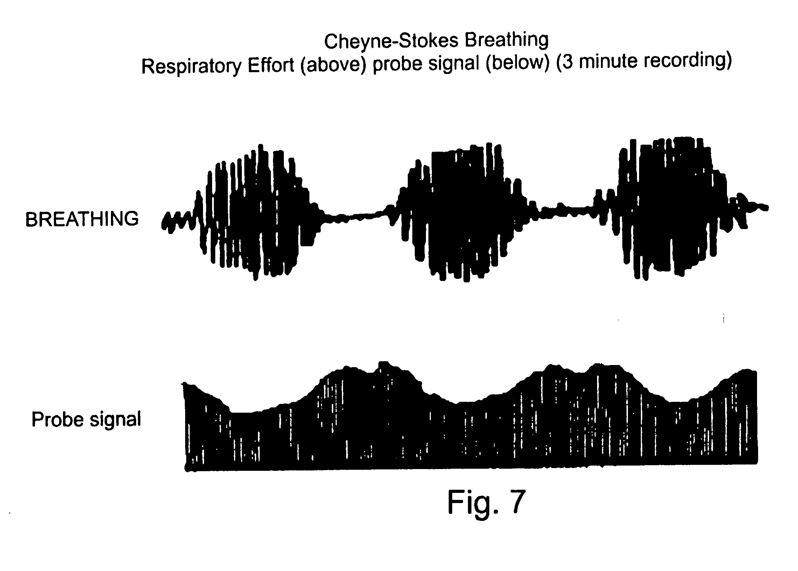

For example a characteristic intermittent breathing pattern, known as "Cheyne-Stokes breathing", occurs in patients suffering from advanced congestive heart failure. In this condition the patients breathing is remarkable in that it consists of alternating crescendo and decrescendo patterns of breathing. The existence of this breathing pattern can be detected by the described finger-probe since a remarkable concordance has been found to exist between the periodic breathing and periodic changes in the amplitude of the finger-probe signal. An example of this is shown in Fig. 7.

It was also found that when the probe output signal, and the oxygen saturation level of the blood, are considered in this condition, the nadirs of the probe output signal coincide with nadirs of blood oxygen saturation levels in some individuals, while it coincides with peak values in others. These differences may be related to cyclic activation of sympathetic nervous activity in response to the accumulating respiratory debt. The probe output signal begins to attenuate at the start of the respiratory crescendo phase in some cases, whereas in other cases, the probe output signal may only begin to increase in amplitude with the onset of the crescendo breathing phase, possibly related to improving cardiac function with the improvement of ventilation.

The probe output signal has also been found to be a highly effective in detecting disordered breathing in sleep based on the circulatory responses to what is primarily a condition of disordered breathing. For example, it is known that apneas terminate in brief arousals, which are associated with tachycardia and elevated blood pressure suggestive of sympathetic activation (Morgan, Crabtree, Puleo. et al., 1996; Pitson and Stradling, 1998).

Thus, using the probe for monitoring the pulse wave in patients with full-blown sleep apnea syndrome revealed that terminations of the apneas are associated with marked attenuation of the pulse wave amplitude and usually with evidence of an increase in pulse rate. The attenuations coincided with the rebreathing phase, maintaining a constant phase relationship with the associated electroencepalographic waking activity. It was found that maximal vasoconstriction was delayed by approximately 7 seconds with respect to the first sign of arousal.

In about 10% of the patients with severe sleep apnea syndrome, using the finger-probe for monitoring the pulse wave during sleep revealed a different picture. In addition to the arousal-related attenuation in the probe output, in these patients the probe output was also profoundly attenuated during the initial stages of the apnea.

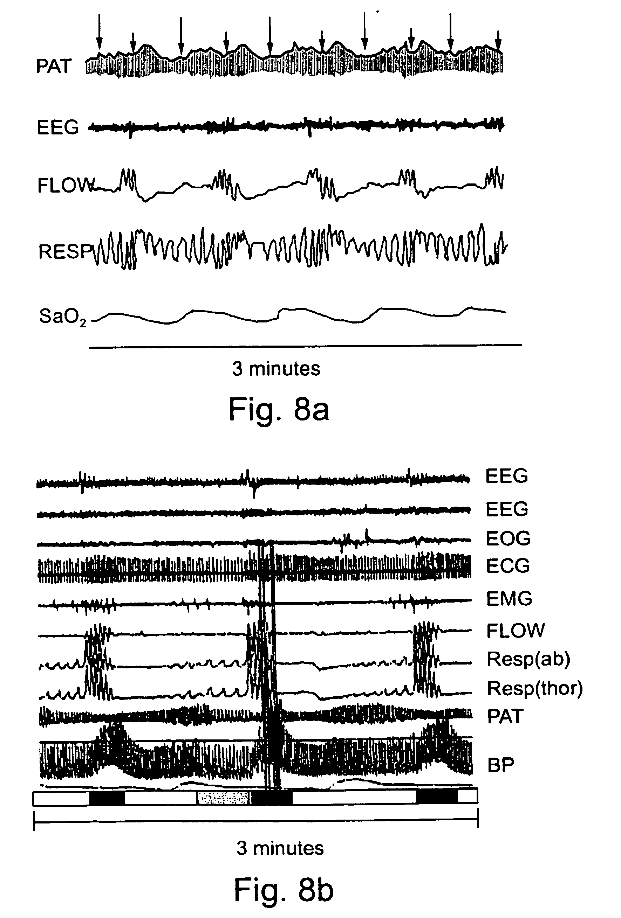

This is exemplified in Figs. 8a and 8b showing that each apnea was associated with two attenuations rather than with one: the first was associated with the onset of the apnea, and the second with the arousal response at the end of the apnea.

In Fig. 8a, the large arrows indicate periods of the probe signal attenuation associated with the apneic periods. The smaller arrows indicate attenuations in the probe amplitude associated with brief arousal which are also associated with renewal of airflow and increased pulse rate. This can be clearly seen in the ECG tracing in Fig. 8b where the distance between the individual ECG signals is noticeably smaller. Similar changes of the pulse rate are seen in the probe signal as well.

In Fig. 8b, the probe attenuation is observed to occur at the termination of apnea as well as during each of the three periods of apnea as in Fig. 8a. During one such apnea, a clear reduction in the arterial blood pressure was observed (area marked in black).

It can clearly be seen that when the attenuation of the probe signal occurs during the brief arousal at the end of the apnea (grey areas), an increase in the pulse rate, and a large increase in blood pressure also occur. The attenuation of the probe signal at such times reflects increased vascular resistance, which is associated with the increased blood pressure.

It is possible to consider the relationship of blood pressure, vascular resistance and cardiac output as being respectively analogous to voltage, resistance and current in Ohm's equation, such that cardiac output is proportional to the ratio between blood pressure and vascular resistance. Thus the increased blood pressure and increased vascular resistance as depicted in the attenuation of the probe signal, could occur in the absence of a change in cardiac output.

In contrast to this situation, a decrease in the probe signal without increased blood pressure could indicate a reduction in cardiac output. A reduction in the probe signal associated with a reduction in blood pressure (as seen in Fig. 8b), would be reflective of an even more substantial decrease in cardiac output.

It is unlikely that the changes in probe amplitude would be related to thermoregulatory affects, or other local regulatory effects, since their duration and repetitive nature are matched to the apnea cycles. It is possible to use the time interval between the appearance of ECG features, such as the QRS complex or the R wave, and features of the pulse-wave such as the peak or start of the upstroke, as an index of pulse transit time (PTT) which is known to be a surrogate of blood pressure. By comparing the PTT during episodes of probe signal attenuation to the intervening periods, it will be possible to differentiate between attenuation episodes associated with increased blood pressure and those not associated with increased blood pressure.

Detrended Fluctuation Analysis (DFA) is a known method for characterizing beat-to-beat variability for ECG heart rate pulses (Peng C-K, Hausdorff JM, Goldberger AL. Fractal mechanisms in neural control: Human heartbeat and gait dynamics in health and disease. In: Walleczek J, ed. Nonlinear Dynamics, Self-Organization, and Biomedicine. Cambridge: Cambridge University Press, 1999). The DFA method is described elsewhere (C.-K.Peng, S. V. Buldyrev, S. Havlin, M. Simons, H.E. Stanley, and A. L. Goldberger, Phys. Rev. E 49, 1685 (1994). The alpha value provided by this method represents an index related to variability.

It has been shown that by applying the DFA method to the signal outputted by the finger-probe described above, both the variability in the beat-to-beat pulse wave amplitude, and the pulse period, can be used for the detection of CHF (using the scaling alpha exponent alpha, which is the result of the DFA method), and that the diagnostic performance can be improved when using both DFA results applied to both amplitude and period of the time series.

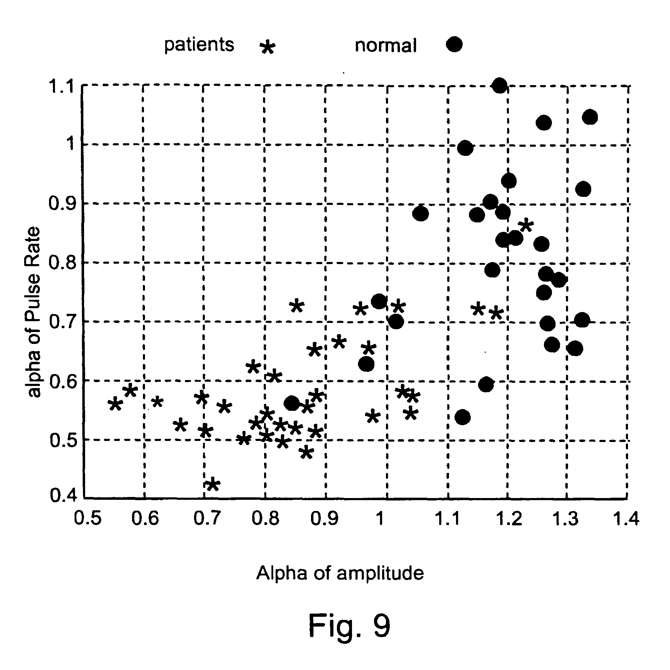

Fig. 9 is a scatter-graph showing alpha values of interbeat period (pulse rate PR) plotted against alpha values of amplitude for CHF patients (stars) and for normal subjects (circles). It can be seen that the separation of the CHF and normal populations is enhanced when both values are considered.

64 subjects (30 normals, 34 CHFs) participated in the study. The area under the curve values for receiver operating characteristics defining the sensitivity and specificity at a range working points of the method for detection of CHF patients are listed in Table 1 below. Also given are corresponding p values.

**Table 1**

| Amp only | PR only | Both Amp & PR |
|---|---|---|
| 0.93 | 0.89 | 0.94 |
| p<0.001 | p<0.001 | pPR=0.155; pAmp=0.003 |

DFA has also previously been used for REM detection in ECG recording (Bunde A., Havlin S., Kantelhardt J.W., Penzel T., Peter J.-H. Peter, and Voigt K. Correlated and uncorrelated regions in heart-rate fluctuations during sleep, Physical Rev Lett 2000, Oct. 23;85(17) 3736-9).

It has been shown that by applying the DFA method to the signal outputted by the finger-probe, either the variability in the beat-to-beat pulse wave amplitude, or the pulse period, can be used for the detection of Rapid Eye Movement (REM) using the scaling exponent (alpha), which is the result of the DFA method. It has also been shown that diagnostic performance is improved when using the combined results of both DFA results from the probe output amplitude (alpha Amp) and period (alpha PR) time series.

25 subjects participated in the study. For each subjects only the last REM and last NREM section (with at least 15 minutes) were analyzed.

The ROC area and the p-value for each test are listed in Table 2 below:

**Table 2**

| Amp only | PR only | Both Amp & PR |
|---|---|---|
| 0.89 | 0.90 | 0.94 |
| p=0.001 | p<0.001 | PPR=.004; pAmp=.008 |

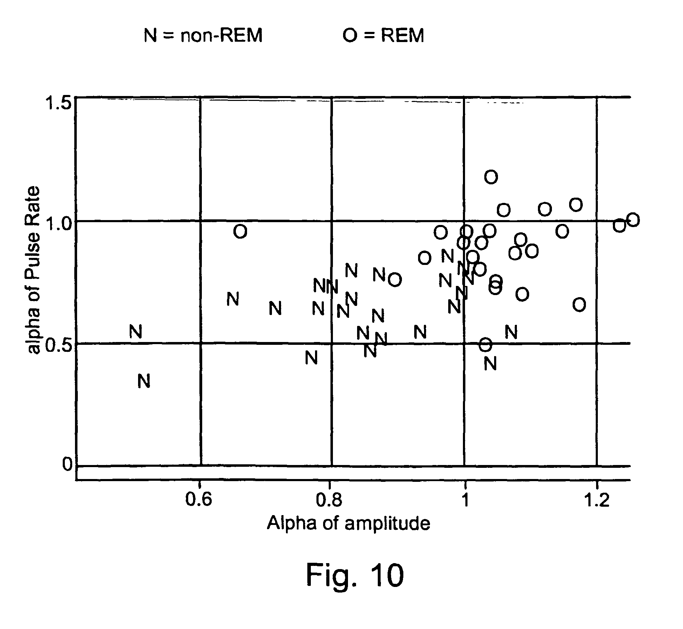

Fig. 10 is a scattergraph showing alphaPR plotted against alphaAmp for the N(non-REM) and O(REM) groups: It is clear that the combined use of both pulse rate alpha value and amplitude alpha values results in a better separation between the REM and non-REM sleep stages than was provided by either value alone.

As indicated earlier, the finger probes of Figs. 1 and 2, which measure changes in the peripheral arterial bed volume, translated into changes in the peripheral arterial tone, have been found to produce the best results in detecting the various sleep state conditions in accordance with the present invention.

## Claims

1. A method of monitoring an individual for determining the occurrence of a particular sleep state condition of the individual, comprising:
applying an external probe to an external surface at a peripheral body location on the individual's body for monitoring the peripheral vascular bed volume of the individual at said peripheral body location by applying a predetermined pressure field to the distal end of said peripheral body location including its distal-most extremity so as to prevent the occurrence of venous pooling within and distal to said peripheral body location, and thereby to produce an output signal from the probe corresponding to changes in the peripheral arterial bed volume at said peripheral body location;
while the individual is in a sleep state, utilizing said probe to monitor changes in the peripheral vascular bed volume of the individual at a monitoring site of said peripheral body location and to produce an output corresponding thereto;
and determining the sleep state condition of the individual according to the output of said external probe; **characterized in that**
the sleep state condition of the individual is determined when a predetermined attenuation, and a predetermined time interval between attenuations, are detected in the output of said external probe.

2. The method according to Claim 1, wherein changes in the individual's pulse rate and/or pulse amplitude are also monitored while the individual is in the sleep state, and such changes are used with said detected attenuations in determining the sleep state condition of the individual.

3. The method according to Claim 1, wherein said pressure field is extended for a distance from said monitoring site towards the heart side of said peripheral body location such as to prevent the occurrence of venous pooling also thereat.

4. The method according to Claim 3, wherein said external probe measures changes in the peripheral arterial tone at said peripheral body location.

5. The method according to Claim 1, wherein said peripheral body location of the individual is a finger, toe or ear lobe of the individual.

6. The method according to Claim 1, wherein said external probe is a volume measuring device.

7. The method according to Claim 1, wherein said external probe is an optical measuring device.

8. The method according to Claim 1, wherein said particular condition is an arousal during the sleep state, and is indicated at least by a predetermined attenuation in the probe output.

9. The method according to Claim 1, wherein said particular condition is an apnea event and is indicated at least by a predetermined attenuation in the probe output.

10. The method according to Claim 1, wherein said particular condition is a hypopnea event and is indicated at least by a predetermined attention in the probe output.

11. The method according to Claim 1, wherein said particular condition is a Cheynes-Stokes breathing pattern and is indicated at least by a predetermined attenuation in the probe output.

12. The method according to Claim 1, wherein said particular condition is a REM (rapid eye movements) and is indicated at least by a predetermined attenuation in the probe output.

13. The method according to Claim 1, wherein the time intervals between attenuations in the probe output are used to distinguish between arousals related to periodical leg movement syndrome (PLMS) and arousals related to disordered breathing events during sleep.

14. The method according to Claim 1, wherein the blood oxygen saturation level of the individual is also monitored to produce an output which is used with the output of said external probe to determine the steep state condition of the individual.

15. The method according to Claim 1, wherein, the oral-nasal airflow of the individual is also monitored to produce an output which is used with the output of said external probe to determine the sleep state condition of the individual.

16. The method according to Claim 1, wherein, the ECG pulse of the individual is also monitored to produce an output which is used with the output of said external probe to determine the sleep state condition of the individual and particularly Pulse Transit Time (PTT).

17. Apparatus for monitoring an individual to detect the occurrence of a particular condition during the sleep state of the individual, comprising:
an external probe to be applied to an external surface at a peripheral body location on the individual's body for monitoring the peripheral vascular bed volume of the individual at said peripheral body location;
said external probe including a pressure applicator for applying a predetermined pressure field to the distal end of said peripheral body location including its distal-most extremity to reduce venous pooling and thereby to produce an output signal from the probe corresponding to changes in the peripheral arterial bed volume at said peripheral body location;
and a signaling device for producing a signal indicating said predetermined sleep state condition when a predetermine change in the output of said probe is detected; **characterized in that**
said signaling device produces a signal indicating said sleep state condition when the output of said probe detects a predetermined attenuation, and a predetermined time interval between attenuations.

18. The apparatus according to Claim 17, wherein said signaling device produces a signal indicating said sleep state condition when a predetermined change in the pulse rate and/or pulse amplitude is also detected.

19. The apparatus according to Claim 17, wherein said probe extends said pressure field for a distance from the signaling device towards the heart side of said peripheral body location such as to prevent the occurrence of venous pooling also thereat.

20. The apparatus according to Claim 17, wherein said external probe measures changes in the peripheral arterial tone at said peripheral body location.

21. The apparatus according to Claim 17, wherein said peripheral body location of the individual is a finger, toe or ear lobe of the individual.

22. The apparatus according to Claim 17, wherein said external probe is a volume measuring device or a device capable of providing an index related to volume.

23. The apparatus according to Claim 17, wherein said external probe is an optical measuring device.

24. The apparatus according to Claim 17, wherein the apparatus further includes a pulse oximeter for monitoring the blood oxygen saturation level of the individual to produce an output which is used by said signaling device with said external probe output to determine the sleep state condition of the individual.

25. The apparatus according to Claim 17, wherein the apparatus further includes an oral-nasal airflow sensor for monitoring the oral-nasal airflow of the individual to produce an output which is used by said signaling device with said external probe output to determine the sleep state condition of the individual.

26. The apparatus according to Claim 17, wherein said apparatus further includes a sleep-wake detector for detecting the sleep/wake status of the individual.

## Patentansprüche

1. Verfahren für das Überwachen eines Individuums für das Bestimmen des Auftretens einer bestimmten Schlafzustandsphase des Individuums, wobei das Verfahren Folgendes umfasst:
Anwenden eines externen Sensors an einer externen Oberfläche an einem peripheren Körperteil auf den Körper des Individuums für das Überwachen des Volumens des Systems peripherer Gefäße des Individuums an dem peripheren Körperteil durch Anwenden eines zuvor bestimmten Druckfelds am distalen Ende des peripheren Körperteils einschließlich dessen äußerste distale Ende, um das Auftreten von venösem Pooling in und distal zu dem peripheren Körperteil zu verhindern und somit ein Ausgabesignal von dem Sensor entsprechend den Veränderungen des Volumens des Systems peripherer Arterien an dem peripheren Körperteil zu erzeugen;
Verwenden des Sensors, während sich das Individuum in einem Schlafzustand befindet, für das Überwachen von Überwachungen des Volumens des Systems peripherer Gefäße des Individuums an einer überwachten Stelle des peripheren Körperteils und das Erzeugen einer dementsprechenden Ausgabe;
und Bestimmen der Schlafzustandsphase des Individuums gemäß der Ausgabe des externen Sensors; **dadurch gekennzeichnet, dass**
die Schlafzustandsphase des Individuums bestimmt wird, wenn eine zuvor bestimmte Abnahme und ein zuvor bestimmtes Zeitintervall zwischen Abnahmen in der Ausgabe des externen Sensors detektiert werden.

2. Verfahren gemäß Anspruch 1, wobei Veränderungen der Pulsfrequenz und/ oder der Pulsamplitude des Individuums ebenfalls überwacht werden, während sich das Individuum in dem Schlafzustand befindet, und solche Veränderungen werden mit den detektierten Abnahmen bei dem Bestimmen der Schlafzustandsphase des Individuums verwendet.

3. Verfahren gemäß Anspruch 1, wobei das Druckfeld auf einen Abstand von der überwachten Stelle hin zur Herzseite des peripheren Körperteils erweitert wird, um das Auftreten von venösem Pooling ebenfalls an der besagten Stelle zu verhindern.

4. Verfahren gemäß Anspruch 3, wobei der externe Sensor Veränderungen des Tonus der peripheren Arterien an dem peripheren Körperteil misst.

5. Verfahren gemäß Anspruch 1, wobei es sich bei dem peripheren Körperteil des Individuums um einen Finger, einen Zeh oder ein Ohrläppchen des Individuums handelt.

6. Verfahren gemäß Anspruch 1, wobei es sich bei dem externen Sensor um eine Vorrichtung für Volumenmessung handelt.

7. Verfahren gemäß Anspruch 1, wobei es sich bei dem externen Sensor um eine Vorrichtung für optische Messung handelt.

8. Verfahren gemäß Anspruch 1, wobei es sich bei der bestimmten Phase um eine Weckreaktion während des Schlafzustands handelt und dieselbe durch zumindest eine zuvor bestimmte Abnahme bei der Sensor-Ausgabe angezeigt wird.

9. Verfahren gemäß Anspruch 1, wobei es sich bei der bestimmten Phase um ein Apnoe-Ereignis handelt und dieselbe durch zumindest eine zuvor bestimmte Abnahme bei der Sensor-Ausgabe angezeigt wird.

10. Verfahren gemäß Anspruch 1, wobei es sich bei der bestimmten Phase um ein Hypopnoe-Ereignis handelt und dieselbe durch zumindest eine zuvor bestimmte Abnahme bei der Sensor-Ausgabe angezeigt wird.

11. Verfahren gemäß Anspruch 1, wobei es sich bei der bestimmten Phase um ein Cheynes-Stokes-Atmungsmuster handelt und dieselbe durch zumindest eine zuvor bestimmte Abnahme bei der Sensor-Ausgabe angezeigt wird.

12. Verfahren gemäß Anspruch 1, wobei es sich der bestimmten Phase um REM (Rapid Eye Movements) handelt und dieselbe durch zumindest eine zuvor bestimmte Abnahme bei der Sensor-Ausgabe angezeigt wird.

13. Verfahren gemäß Anspruch 1, wobei die Zeitintervalle zwischen Abnahmen bei der Sensor-Ausgabe verwendet werden, um zwischen Weckreaktionen in Zusammenhang mit periodischen Beinbewegungen im Schlaf (PLMS, *Periodic Limb Movements in Sleep*) und Weckreaktionen in Zusammenhang mit Atemstörungen während des Schlafes zu unterscheiden.

14. Verfahren gemäß Anspruch 1, wobei der Wert der Sauerstoffsättigung im Blut des Individuums ebenfalls überwacht wird, um eine Ausgabe zu erzeugen, die mit der Ausgabe des externen Sensors für das Bestimmen der Schlafzustandsphase des Individuums verwendet wird.

15. Verfahren gemäß Anspruch 1, wobei der oral-nasale Luftstrom des Individuums ebenfalls überwacht wird, um eine Ausgabe zu erzeugen, die mit der Ausgabe des externen Sensors für das Bestimmen der Schlafzustandsphase des Individuums verwendet wird.

16. Verfahren gemäß Anspruch 1, wobei der EKG-Puls des Individuums ebenfalls überwacht wird, um eine Ausgabe zu erzeugen, die mit der Ausgabe des externen Sensors für das Bestimmen der Schlafzustandsphase des Individuums und insbesondere der Puls-Transit-Zeit (PTT) verwendet wird.

17. Vorrichtung für das Überwachen eines Individuums, um das Auftreten einer bestimmten Phase während des Schlafzustands des Individuums zu detektieren, wobei die Vorrichtung Folgendes umfasst:
einen externen Sensor, die an einer externen Oberfläche an einem peripheren Körperteil auf den Körper des Individuums für das Überwachen des Volumens des Systems peripherer Gefäße des Individuums an dem peripheren Körperteil angewendet werden soll;
wobei der externe Sensor einen Druckapplikator für das Anwenden eines zuvor bestimmten Druckfelds auf das distale Ende des peripheren Körperteils einschließlich dem äußersten distalen Ende einschließt, um venöses Pooling zu reduzieren und somit ein Ausgabe-Signal von dem Sensor entsprechend den Veränderungen des Volumens des Systems peripherer Arterien an dem Körperteil zu erzeugen;
und eine Signalisierungsvorrichtung für das Erzeugen eines Signals, das die zuvor bestimmte Schlafzustandsphase anzeigt, wenn eine zuvor bestimmte Veränderung bei der Ausgabe des Sensors detektiert wird, **dadurch gekennzeichnet, dass**
die Signalisierungsvorrichtung ein Signal erzeugt, das die Schlafzustandsphase anzeigt, wenn die Ausgabe des Sensors eine zuvor bestimmte Abnahme und ein zuvor bestimmtes Zeitintervall zwischen Abnahmen detektiert.

18. Vorrichtung gemäß Anspruch 17, wobei die Signalisierungsvorrichtung ein Signal erzeugt, das die Schlafzustandsphase anzeigt, wenn ebenfalls eine zuvor bestimmte Veränderung der Pulsfrequenz und/ oder der Pulsamplitude detektiert wird.

19. Vorrichtung gemäß Anspruch 17, wobei der Sensor das Druckfeld auf einen Abstand von der Signalisierungsvorrichtung hin zur Herzseite des peripheren Körperteils erweitert, um ebenfalls an dieser Stelle ein Auftreten von venösem Pooling zu verhindern.

20. Vorrichtung gemäß Anspruch 17, wobei der externe Sensor Änderungen des Tonus peripherer Arterien an dem peripheren Körperteil misst.

21. Vorrichtung gemäß Anspruch 17, wobei es sich bei dem peripheren Körperteil des Individuums um einen Finger, einen Zeh oder ein Ohrläppchen des Individuums handelt.

22. Vorrichtung gemäß Anspruch 17, wobei es sich bei dem externen Sensor um eine Vorrichtung für Volumenmessung oder eine Vorrichtung, die einen Volumen-bezogenen Index bereitstellen kann, handelt.

23. Vorrichtung gemäß Anspruch 17, wobei es sich bei dem externen Sensor um eine Vorrichtung für optische Messung handelt.

24. Vorrichtung gemäß Anspruch 17, wobei die Vorrichtung ferner ein Puls-Oximeter für das Überwachen des Werts der Sauerstoffsättigung im Blut des Individuums einschließt, um eine Ausgabe zu erzeugen, die von der Signalisierungsvorrichtung mit der Ausgabe des externen Sensors für das Bestimmen der Schlafzustandsphase des Individuums verwendet wird.

25. Vorrichtung gemäß Anspruch 17, wobei die Vorrichtung ferner einen Messfühler für oral-nasalen Luftstrom für das Überwachen des oral-nasalen Luftstroms des Individuums einschließt, um eine Ausgabe zu erzeugen, die von der Signalisierungsvorrichtung mit der Ausgabe des externen Sensors für das Bestimmen der Schlafzustandsphase des Individuums verwendet wird.

26. Vorrichtung gemäß Anspruch 17, wobei die Vorrichtung ferner einen Schlaf-Wach-Detektor für das Detektieren von Schlaf/ Wachstatus des Individuums einschließt.

## Revendications

1. Procédé de surveillance d'un individu pour déterminer l'apparition d'une condition d'état de sommeil particulier de l'individu, comprenant :
- l'application d'une sonde externe à une surface externe à un emplacement du corps périphérique sur le corps de l'individu pour surveiller le volume de lit vasculaire périphérique de l'individu audit emplacement de corps périphérique par application d'un champ de pression prédéterminé à l'extrémité distale dudit emplacement de corps périphérique comprenant son extrémité la plus distale de façon à empêcher l'apparition d'une accumulation veineuse à l'intérieur et de façon distale par rapport audit emplacement de corps périphérique, et pour produire ainsi un signal de sortie à partir de la sonde correspondant à des changements dans le volume de lit artériel périphérique audit emplacement de corps périphérique ;
- alors que l'individu est dans un état de sommeil, l'utilisation de ladite sonde pour surveiller des changements dans le volume de lit vasculaire périphérique de l'individu à un site de surveillance dudit emplacement de corps périphérique et pour produire une sortie correspondant à celle-ci ; et
- la détermination de la condition d'état de sommeil de l'individu conformément à la sortie de ladite sonde externe ; **caractérisé par le fait que** la condition d'état de sommeil de l'individu est déterminée lorsqu'une atténuation prédéterminée et un intervalle de temps prédéterminé entre les atténuations, sont détectés dans la sortie de ladite sonde externe.

2. Procédé selon la revendication 1, dans lequel des changements dans la fréquence du pouls et/ou l'amplitude des pulsations de l'individu sont également surveillés alors que l'individu est dans l'état de sommeil, et de tels changements sont utilisés avec lesdites atténuations détectées dans la détermination de la condition d'état de sommeil de l'individu.

3. Procédé selon la revendication 1, dans lequel ledit champ de pression est étendu sur une distance à partir dudit site de surveillance vers le côté coeur dudit emplacement de corps périphérique de façon à empêcher l'apparition d'une accumulation veineuse également à cet endroit.

4. Procédé selon la revendication 3, dans lequel ladite sonde externe mesure des changements dans le tonus artériel périphérique audit emplacement de corps périphérique.

5. Procédé selon la revendication 1, dans lequel ledit emplacement de corps périphérique de l'individu est un doigt, un orteil ou un lobe d'oreille de l'individu.

6. Procédé selon la revendication 1, dans lequel ladite sonde externe est un dispositif de mesure de volume.

7. Procédé selon la revendication 1, dans lequel ladite sonde externe est un dispositif de mesure optique.

8. Procédé selon la revendication 1, dans lequel ladite condition particulière est un réveil pendant l'état de sommeil et est indiquée au moins par une atténuation prédéterminée dans la sortie de la sonde.

9. Procédé selon la revendication 1, dans lequel ladite condition particulière est un événement d'apnée et est indiquée au moins par une atténuation prédéterminée dans la sortie de la sonde.

10. Procédé selon la revendication 1, dans lequel ladite condition particulière est un événement d'hypopnée et est indiquée au moins par une attention prédéterminée dans la sortie de sonde.

11. Procédé selon la revendication 1, dans lequel ladite condition particulière est un motif de respiration de Cheynes-Stokes et est indiquée au moins par une atténuation prédéterminée dans la sortie de sonde.

12. Procédé selon la revendication 1, dans lequel ladite condition particulière est une période de mouvements oculaires survenant au cours du sommeil (mouvements oculaires rapides) et est indiquée au moins par une atténuation prédéterminée dans la sortie de sonde.

13. Procédé selon la revendication 1, dans lequel les intervalles de temps entre les atténuations de la sortie de sonde sont utilisés pour distinguer entre des réveils se rapportant à un syndrome de mouvements périodiques des jambes (PLMS) et des réveils se rapportant à des événements de respiration désordonnés pendant le sommeil.

14. Procédé selon la revendication 1, dans lequel le taux de saturation d'oxygène du sang de l'individu est également surveillé pour produire une sortie qui est utilisée avec la sortie de ladite sonde externe pour déterminer la condition d'état de sommeil de l'individu.

15. Procédé selon la revendication 1, dans lequel l'écoulement de l'air oral-nasal de l'individu est également surveillé pour produire une sortie qui est utilisée avec la sortie de ladite sonde externe pour déterminer la condition d'état de sommeil de l'individu.

16. Procédé selon la revendication 1, dans lequel l'impulsion d'ECG de l'individu est également surveillée pour produire une sortie qui est utilisée avec la sortie de ladite sonde externe pour déterminer la condition d'état de sommeil de l'individu et en particulier le temps de transit d'impulsion (PTT).

17. Appareil pour surveiller un individu pour détecter l'apparition d'une condition particulière pendant l'état de sommeil de l'individu, comprenant :
- une sonde externe devant être appliquée à une surface externe à un emplacement de corps périphérique sur le corps de l'individu pour surveiller le volume de lit vasculaire périphérique de l'individu audit emplacement de corps périphérique ;
- ladite sonde externe comprenant un applicateur de pression pour appliquer un champ de pression prédéterminé à l'extrémité distale dudit emplacement de corps périphérique comprenant son extrémité la plus distale pour réduire une accumulation veineuse et par là pour produire un signal de sortie à partir de la sonde correspondant à des changements dans le volume de lit artériel périphérique audit emplacement de corps périphérique ; et
- un dispositif de signalisation pour produire un signal indiquant ladite condition d'état de sommeil prédéterminée lorsqu'un changement prédéterminé dans la sortie de la sonde est détecté ; **caractérisé par le fait que** ledit dispositif de signalisation produit un signal indiquant ladite condition d'état de sommeil lorsque la sortie de ladite sonde détecte une atténuation prédéterminée et un intervalle de temps prédéterminé entre des atténuations.

18. Appareil selon la revendication 17, dans lequel ledit dispositif de signalisation produit un signal indiquant ladite condition d'état de sommeil lorsqu'un changement prédéterminé dans la fréquence du pouls et/ou l'amplitude des pulsations est également détecté.

19. Appareil selon la revendication 17, dans lequel ladite sonde étend ledit champ de pression sur une distance à partir du dispositif de signalisation vers le côté coeur dudit emplacement de corps périphérique de façon à empêcher l'apparition d'une accumulation veineuse également à cet endroit.

20. Appareil selon la revendication 17, dans lequel ladite sonde externe mesure des changements dans le tonus artériel périphérique audit emplacement de corps périphérique.

21. Appareil selon la revendication 17, dans lequel ledit emplacement de corps périphérique de l'individu est un doigt, un orteil ou un lobe d'oreille de l'individu.

22. Appareil selon la revendication 17, dans lequel ladite sonde externe est un dispositif de mesure de volume ou un dispositif capable de fournir un indice se rapportant au volume.

23. Appareil selon la revendication 17, dans lequel ladite sonde externe est un dispositif de mesure optique.

24. Appareil selon la revendication 17, dans lequel l'appareil comprend en outre un oxymètre de pouls pour surveiller le taux de saturation d'oxygène du sang de l'individu pour produire une sortie qui est utilisée par ledit dispositif de signalisation avec ladite sortie de sonde externe pour déterminer la condition d'état de sommeil de l'individu.

25. Appareil selon la revendication 17, dans lequel l'appareil comprend en outre un détecteur de l'écoulement d'air oral-nasal pour surveiller l'écoulement d'air oral-nasal de l'individu pour produire une sortie qui est utilisée par ledit dispositif de signalisation avec ladite sortie de sonde externe pour déterminer la condition d'état de sommeil de l'individu.

26. Appareil selon la revendication 17, dans lequel ledit appareil comprend en outre un détecteur sommeil-éveil pour détecter l'état de sommeil/éveil de l'individu.
